# EUROPÄISCHE PATENTSCHRIFT

(11) **EP 1 529 769 B1**
(45) Veröffentlichungstag und Bekanntmachung des Hinweises auf die Patenterteilung: **11.04.2007**
(21) Anmeldenummer: 04025483.1
(22) Anmeldetag: 27.10.2004
(51) Int. Cl.: C07C 45/50, C07C 47/347, C07C 47/445

(54) **Verfahren zur Herstellung von TCD-Dialdehyd**
Process for the preparation of TCD-dialdehyde
Procédé de préparation de TCD-dialdehyde

(30) Priorität: 08.11.2003 DE 10352261
(43) Veröffentlichungstag der Anmeldung: 11.05.2005
(73) Patentinhaber: Celanese Chemicals Europe GmbH, 61476 Kronberg (DE)
(72) Erfinder: Lappe, Peter, Dr., 46539 Dinslaken (DE); Springer, Helmut, 46539 Dinslaken (DE); Lukas, Rainer, Dr., 45133 Essen (DE)

(56) Entgegenhaltungen:
- EP-A- 0 186 075
- EP-A- 0 348 832
- EP-A- 0 811 424
- DE-A- 2 928 313
- DATABASE CAPLUS CHEMICAL ABSTRACTS SERVICE, COLUMBUS, OHIO, US; XP002306410 gefunden im STN Database accession no. 1981:514918 & JP 56 030938 A (MITSUBISHI PETROCHEMICAL CO LTD) 28. März 1981 (1981-03-28)

## Beschreibung

Die vorliegende Erfindung betrifft ein Verfahren zur Herstellung von TCD-Dialdehyd {3(4),8(9)-Bis(formyl)-tricyclo[5.2.1.0^{2.6}]decan} aus Dicyclopentadien (DCP).

Das durch Dimerisierung von Cyclopentadien leicht zugängliche und auch großtechnisch hergestellte Dicyclopentadien (DCP) lässt sich zu anwendungstechnisch wichtigen Verbindungen umsetzen, denen das Tricyclodecan-Gerüst besondere Eigenschaften verleiht. Die vom DCP-abgeleiteten Verbindungen mit Tricyclodecan-Struktur werden in der Literatur häufig unterschiedlich bezeichnet. In Anlehnung an die aus Chemiker-Zeitung, 98, 1974, Seiten 70 bis 76 bekannte Nomenklatur für DCP-Derivate, wird auch im folgenden die auf dem Tricyclodecan-Gerüst, auch TCD-Gerüst genannt, aufbauende Nomenklatur verwendet.

Insbesondere die Hydroformylierung von DCP liefert interessante TCD-Aldehyde, wie 8(9)-Formyl-tricyclo[5.2.1 .0^{2.6}]dec-3-en, auch als TCD-Monenal bezeichnet, oder 3(4),8(9)-Bisformyl-tricyclo[5.2.1.0^{2.6}]decan, auch als TCD-Dialdehyd bezeichnet, die zu wichtigen Zwischenprodukten weiterverarbeitet werden. So führt die reduktive Aminierung von TCD-Dialdehyd zu TCD-Diamin {3(4),8(9)-Bis(aminomethyl)-tricyclo[5.2.1.0^{2.6}]decan}, das als wertvolles Zwischenprodukt in zahlreichen technisch ausgeübten Synthesen Verwendung findet.

Beispielsweise wird TCD-Diamin zur Herstellung lichtbeständiger Polyurethansysteme gemäß DE 28 19 980 oder zur Herstellung hitzehärtbarer Beschichtungsmaterialien nach EP 59 962 verwendet.

Die Hydrierung von TCD-Dialdehyd führt zum TCD-Alkohol DM {3(4),8(9)-Dihydroxymethyl-tricyclo[5.2.1.0^{2.6}]decan}, das ebenfalls eine große wirtschaftliche Bedeutung besitzt, beispielsweise als Bestandteil von bei Sauerstoffausschluss erhärtbaren Acrylsäureesterklebstoffen (EP 23 686).

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Co als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Wegen der Gefahr der Retro-Diels-Alder-Reaktion bei den Temperaturen der Oxo-Synthese und der damit verbundenen Freisetzung von Cyclopentadien, das zur Komplexbildung gegenüber Übergangsmetallen befähigt ist und das die Aktivität der eingesetzten Katalysatoren mindern kann, muss die Hydroformylierung unter besonderen Bedingungen ablaufen. Es erwies sich als vorteilhaft, den früher üblichen Co-Katalysator durch Rhodium zu ersetzen, das eine hohe Selektivität der Umsetzung zu Aldehyden erreichen lässt und die Hydroformylierung bei Bedingungen erlaubt, bei denen das Ausmaß der Retro-Diels-Alder-Spaltung geringer ist. Eine zusammenfassende Darstellung über die Hydroformylierung von Dicyclopentadien findet sich in Chemiker-Zeitung 98, 1974, 70-76.

Im Stand der Technik wird auf die thermische Labilität der TCD-Aldehyde hingewiesen, die bei der destillativen Aufarbeitung des rohen Hydroformylierungsgemischs zu hohen Produktverlusten führt. Aufgrund dieser bekannten thermischen Instabilität der TCD-Aldehyde und insbesondere der von TCD-Dialdehyd werden diese Aldehyde zumeist nicht rein dargestellt, sondern in ihren Gemischen mit den Nebenprodukten der Oxo-Synthese weiter verarbeitet (Chemikerzeitung, 98 (2), 1974, Seite 72).

In der Literatur sind ferner Extraktionsverfahren zur Aufarbeitung des Rohgemisches aus der DCP-Hydroformylierung bekannt. Gemäß der EP 1 065 194 erfolgt die Aufarbeitung des Hydroformylierungsprodukts durch eine mehrstufige Extraktion unter Verwendung von mehrwertigen Alkoholen wie z.B. Ethylenglykol, wobei der Zusatz von tertiären Aminen empfohlen wird. Nach der Extraktion liegt das Oxorohprodukt vorwiegend in der Alkoholphase vor, während sich geringe Anteile an Mono- und Dialdehyd sowie die Hauptmenge an Rhodium und Phosphin-Liganden in der Kohlenwasserstoffphase befinden. Es wird darauf hingewiesen, dass die Extraktion in absoluter Abwesenheit von Sauerstoff erfolgen muss. Der Einsatz von Extraktionsmitteln unter Zusatz von tertiären Aminen sowie die absolute Notwendigkeit der Abwesenheit von Sauerstoff erschweren die technische Durchführung dieses Verfahrens und beinhalten das Risiko der Verunreinigung mit Amin-Spuren.

Nach US 5,138,101 verwendet man zur Extraktion ein Methanol/Wasser-Gemisch, wobei die TCD-Aldehyde in die polare, alkoholische Phase übergehen.

In der JP 58 021 638 wird ein Verfahren zur Abtrennung von Aldehyden, erhalten durch Hydroformylierung von nicht-konjugierten Diolefinen, beschrieben. Dabei wird das Oxorohprodukt mit einer wässrigen Lösung von Alkalihydrogensulfit behandelt und anschließend von der Rh-Katalysator enthaltenden organischen Phase abgetrennt. Dieses Trennverfahren ist aus wirtschaftlichen und technischen Gründen nicht vorteilhaft. Das Aldehyd-Bisulfit-Addukt muss durch aufwendige Maßnahmen wieder in den Aldehyd zurückgespalten werden. Zudem beinhaltet das Verfahren den Einsatz von schwefelhaltigen Verbindungen und birgt somit das Risiko zu entsprechenden Verunreinigungen im abgetrennten Aldehyd. Schließlich entstehen bei diesem Prozess beachtliche Mengen an Abwasser, die zu einer beträchtlichen Umweltbelastung führen.

JP56030938 offenbart einen Prozess, in dem Dicyclopentadien in der Gegenwart von HRh(CO)(PPh₃)₃ in einem Schritt zu TCD-Dialdehyd in homogener Phase hydroformyliert wird, mit 100% Umsatz von Dicyclopentadien und 92.2% Selektivität zu TCD-Dialdehyd.

Die bekannten Verfahren zur Herstellung von TCD-Dialdehyd durch Hydroformylierung von Dicyclopentadien stellen gereinigten TCD-Dialdehyd entweder in nur wirtschaftlich unbefriedigenden Ausbeuten und Selektivitäten bereit oder erfordern ein aufwendiges Extraktionsverfahren. Die Bereitstellung von gereinigtem TCD-Dialdehyd ist jedoch von wirtschaftlichem Interesse. So wird beispielsweise der TCD-Dialdehyd auf dem Riechstoffsektor (DE 19 817 044), als Monomer zur Herstellung von Polyestern (JP 11 080 068) und zur Herstellung von Bakteriziden benötigt. Es besteht daher ein Bedarf nach einem Verfahren, das TCD-Dialdehyd möglichst einfach und kostengünstig in gereinigter Form zugänglich macht.

Die Erfindung besteht daher in einem Verfahren zur Herstellung von 3(4),8(9)-Bisformyl-tricyclo[5.2.1.0^{2.6}]decan durch Hydroformylierung von Dicyclopentadien. Es ist dadurch gekennzeichnet, dass man Dicyclopentadien in einer ersten Hydroformylierungsstufe in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas zum 8(9)-Formyl-tricyclo[5.2.1.0^{2.6}]dec-3-en umsetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Übergangsmetallverbindungen der Gruppe VIII des Periodensystems der Elemente bei Temperaturen von 70 bis 140°C und Drücken von 5 bis 35 MPa durch Umsetzung mit Synthesegas in 3(4),8(9)-Bisformyltricyclo[5.2.1.0^{2.6}]decan überführt und das so erhaltene 3(4),8(9)-Bisformyltricyclo[5.2.1.0^{2.6}]decan anschließend destilliert.

Charakteristisch für das erfindungsgemäße Hydroformylierungsverfahren von Dicyclopentadien ist die zweistufige Reaktionsführung, wobei in der ersten Stufe nach dem heterogenen Zweiphasenprozess in Gegenwart einer wässrigen Katalysatorlösung gearbeitet wird und das Reaktionsprodukt der ersten Stufe, enthaltend überwiegend TCD-Monoaldehyd und geringe Mengen an nicht umgesetzten DCP, ohne weitere Reinigung in einer zweiten Stufe nach Katalysatorzusatz in einem homogenen Reaktionsmedium zum TCD-Dialdehyd umgesetzt wird. Durch diese Art der Reaktionsführung erfolgt in der ersten Reaktionsstufe eine sehr selektive Hydroformylierung der im Sechsring des TCD-Gerüstes vorhandenen Doppelbindung zu TCD-Monoaldehyd, der häufig auch als TCD-Monenal {8(9)-Formyl-tricyclo[5.2.1.0^{2.6}]dec-3-en} bezeichnet wird.

Überraschenderweise zeigt sich, dass das Reaktionsprodukt der ersten Hydroformylierungsstufe nach Abtrennung der wässrigen Katalysatorphase ohne weitere Reinigung in einem homogenen organischen Medium nach Katalysatorzusatz zum TCD-Dialdehyd hydroformyliert werden kann, obwohl die das Wertprodukt enthaltende organische Phase homogen gelöste und analytisch nachweisbare Mengen an Phosphor- und Schwefel-Spalt- und Abbauprodukten enthält, die als Katalysatorgifte in der Oxo-Synthese bekannt sind.

Nach "New Synthesis with Carbon Monoxide" (Edited by J. Falbe, Springer-Verlag 1980, Reactivity and Structure Concepts in Organic Chemistry, Vol. 11, Seite 73) sind bei der rhodiumkatalysierten Hydroformylierung zahlreiche Katalysatorgifte bekannt. Neben Halogen, Acetylenen und Carbonsäuren wird vor allem auch auf Schwefel hingewiesen. Bereits geringe Mengen dieser Katalysatogifte bewirken eine drastische Deaktivierung des Hydroformylierungskatalysators.

Überraschenderweise zeigte sich ebenfalls, dass das in der ersten Stufe nicht vollständig umgesetzte Dicyclopentadien in der zweiten Hydroformylierungsstufe ohne nennenswerte Bildung von hochsiedenden Nebenprodukten vollständig in den TCD-Dialdehyd überführt werden kann. Daraus ergibt sich die vorteilhafte Möglichkeit einer DCP-Teilumsatzfahrweise in der ersten Hydroformylierungsstufe.

Eine destillative Reinigung von TCD-Monenal aus der abgetrennten organischen Phase der ersten Hydroformylierungsstufe ist jedoch nicht ausgeschlossen. Diese Arbeitsweise bedarf jedoch eines zusätzlichen Destillationsschrittes und führt, wenn auch zu nur geringen Destillationsverlusten. Die gezielte Herstellung von TCD-Monenal aus Dicyclopentadien unter Verwendung einer wässrigen Katalysatorlösung sowie seine destillative Reinigung ist aus EP-B1-0 186 075 bekannt.

Die erste Reaktionsstufe des neuen Verfahrens führt man als heterogene Reaktion im einem Zweiphasensystem durch, eine Umsetzung, die z.B. in der DE-B-26 27 354 beschrieben ist. Dieser Prozess ist charakterisiert durch das Vorliegen einer organischen Phase, die das olefinische Ausgangsmaterial und das Reaktionsprodukt enthält, und einer wässrigen Phase, in der der Katalysator gelöst ist. Als Katalysatoren werden wasserlösliche Rhodium-Komplexverbindungen eingesetzt, die wasserlösliche organischen Phosphor(III)-Verbindungen als Liganden enthalten. Beispiele für wasserlösliche Phosphor(III)-Verbindungen, die mit Rhodium Komplexverbindungen bilden, sind Triarylphosphine, Trialkylphosphine, gemischte aliphatische-aromatische Phosphine und arylierte bzw. alkylierte Diphosphine, deren organische Reste Sulfonsäuregruppen oder Carboxylgruppen enthalten. Ihre Herstellung und Anwendung ist z.B. aus DE-B 26 27 354, EP-B1-0 103 810, EP-B1-0 163 234 und EP-A1-0 571 819 bekannt. Weitere Gruppen geeigneter Verbindungen sind sulfonierte oder carboxylierte organische Phosphite sowie heterocyclische Verbindungen des dreiwertigen Phosphors, die z.B. aus EP-A1-0 575 785 und EP-A1-0 646 588 bekannt sind.

Als sulfonierte Arylphosphine eignen sich in dem erfindungsgemäßen Verfahren sulfonierte Triarylphosphine der allgemeinen Formel (I) 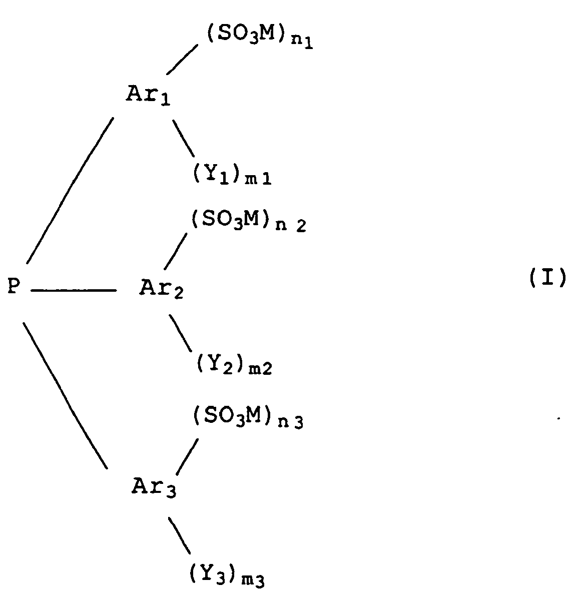 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen zählen bevorzugt solche Triarylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Als sulfonierte Arylphosphine eignen sich ebenfalls sulfonierte Diphosphine der allgemeinen Formeln (II) oder (III)

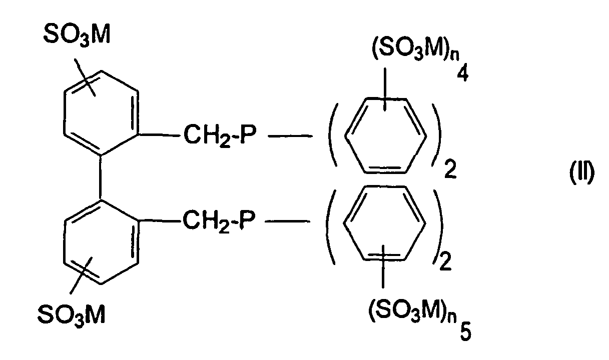

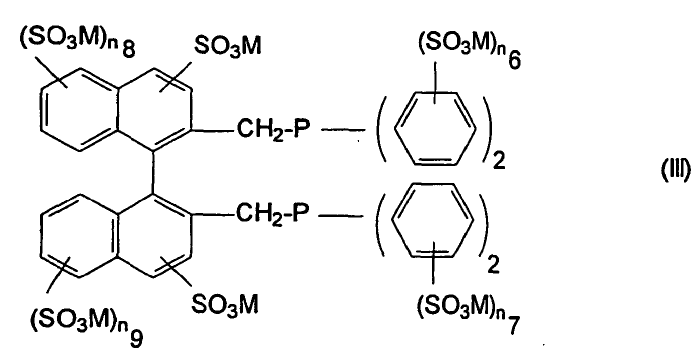

Diese Diphosphine der allgemeinen Formeln (II) und (III) sind aus W098/30526 bekannt.

In (II) steht ein jedes n₄ und n₅ unabhängig voneinander für 0 oder 1, wobei die Verbindung der Formel (II) bis sechs -SO₃M-Gruppen enthält.

In (III) steht ein jedes n₆, n₇, n₈ und n₉ unabhängig voneinander für 0 oder 1, wobei die Verbindung der Formel (III) vier bis acht -SO₃M-Gruppen enthält.

Aufgrund der Herstellung durch Sulfonierung der entsprechenden Diphosphine der Formeln (IIa) und (IIIa), die keine -SO₃M-Gruppen enthalten, 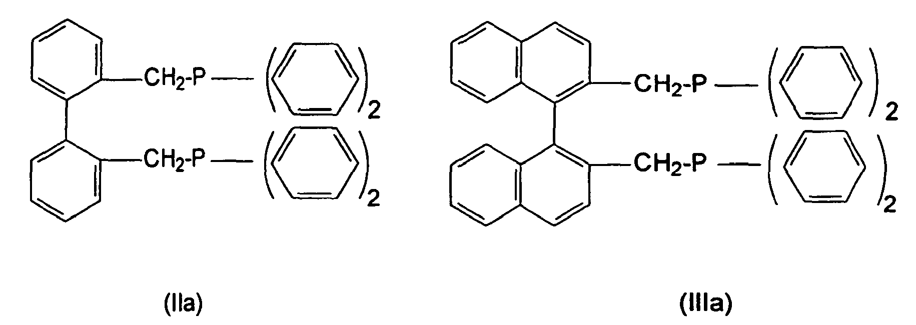 erhält man üblicherweise Gemische von Verbindungen (II) und (III) mit unterschiedlicher Anzahl von -SO₃M-Gruppen. So enthält eine Verbindung der Formeln (II) oder (III), die beispielsweise drei -SO₃M-Gruppen enthält, auch Verbindungen mit lediglich zwei -SO₃M-Gruppen aber auch Verbindungen mit vier oder fünf -SO₃M-Gruppen. Eine Verbindung der Formeln (II) oder (III) mit beispielsweise fünf -SO₃M-Gruppen enthält üblicherweise auch Verbindungen mit lediglich drei oder vier -SO₃M-Gruppen aber auch Verbindungen mit sechs oder sieben -SO₃M-Gruppen.

Verbindungen der Formel (II) besitzen maximal sechs -SO₃M-Gruppen, während Verbindungen der Formel (III) maximal acht -SO₃M-Gruppen aufweisen.

Aus diesem Grund gelangen in der Regel Mischungen von Verbindungen der Formeln (II) und (III) mit unterschiedlicher Anzahl von -SO₃M-Gruppen zum Einsatz.

In den Formeln (II) und (III) steht M für Ammonium, ein einwertiges Metall oder das Äquivalent eines mehrwertigen Metalls, insbesondere für Natrium, Kalium, Calcium oder Barium.

Besonders vorteilhaft ist der Einsatz wasserlöslicher Komplexverbindungen des Rhodiums, obwohl die Verwendung anderer katalytisch aktiver Übergangsmetallverbindungen der Gruppe VIII des Periodensystems der Elemente nicht ausgeschlossen wird. So kann man in der ersten Hydroformylierungsstufe auch wasserlösliche Komplexverbindungen von Kobalt, Iridium, Nickel, Palladium, Platin, Eisen oder Ruthenium verwenden, und besonders wasserlösliche Komplexverbindungen des Kobalts, Iridiums und Platins haben sich als wirksame Hydroformylierungskatalysatoren erwiesen.

Die Bedingungen, unter denen die Umsetzung in der ersten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 70 bis 150°C durch. Bevorzugt hält man Temperaturen von 100 bis 150°C und insbesondere von 110 bis 140°C ein. Der Gesamtdruck erstreckt sich über einen Bereich von 0,5 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Rhodium-Konzentration beträgt 20 bis 1000 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphor-Liganden, d.h. Ligand, der mit Rhodium keine komplexe Bindung eingegangen ist. Je mol Rhodium wendet man bevorzugt 10 bis 300 mol Phosphor in Form einer wasserlöslichen organischen Phosphorverbindung an. Besonders bewährt haben sich molare Verhältnisse von Rhodium zu Phosphor im Bereich von 1:50 bis 1:150. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Wird ein anderes Übergangsmetall der Guppe VIII des Periodensystems der Elemente.als katalytisch aktives Metall eingesetzt, so bewegt sich die Konzentration an Übergangsmetall sowie das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Man bildet den Katalysator üblicherweise aus den Komponenten Übergangsmetall oder Übergangsmetallverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Dicylopentadien kann als solches oder in Lösung der Hydroformylierung zugeführt werden. Geeignete Lösungsmittel sind wasserunlösliche Ketone, Dialkylether, aliphatische Nitrile, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Um den Umsatz je Zeiteinheit von Dicyclopentadien, das in der wässrigen Katalysatorlösung nur wenig löslich sind, zu erhöhen, kann es sich empfehlen, dieser Lösung ein Phasentransferreagenz (Lösungsvermittler) zuzusetzen. Er verändert die physikalischen Eigenschaften der Grenzflächen zwischen den beiden flüssigen Phasen und erleichtert den Übergang des organischen Reaktanten in die wässrige Katalysatorlösung.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren, vorzugsweise solcher mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wässriger Lösung nicht in lonen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. aus EP-B1-0 157 316 bekannt.

Es können auch Rhodiumkomplexverbindungen eingesetzt werden, die gleichzeitig Katalysator und Phasentransferreagenz sind. Eine solche Arbeitsweise ist z.B. Gegenstand der EP-B1-0 163 234.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der ersten Hydroformylierungsstufe wird in einem Phasentrenner in die organische Produktphase und in die wässrige Katalysatorlösung aufgetrennt. Es hat sich als zweckmäßig erwiesen, die Katalysatorlösung im Kreis zu führen. Die rohe organische Produktphase wird ohne weitere Reinigungsschritte der zweiten Hydroformylierungsstufe zugeführt. Eine zwischengeschaltete destillative Reinigung des Reaktionsproduktes der ersten Hydroformylierungsstufe kann aber gegebenenfalls auch durchgeführt werden.

Die zweite Hydroformylierungsstufe des neuen Verfahrens führt man in einem homogenen Reaktionssystem durch. Der Begriff homogenes Reaktionssystem steht für eine im wesentlichen aus Lösungsmittel, falls in der ersten Stufe und/oder in der zweiten Reaktionsstufe zugesetzt, Katalysator, unumgesetztes Dicyclopentadien und TCD-Monenal zusammengesetzte homogene Lösung. Gegebenenfalls kann sich ein Lösungsmittelzusatz in der zweiten Reaktionsstufe als zweckmäßig erweisen. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Ein Lösungsmittelzusatz in der zweiten, wie auch in der ersten Hydroformylierungsstufe, ist jedoch nicht zwingend erforderlich.

Als Katalysatoren in der zweiten Hydroformylierungsstufe verwendet man Übergangsmetallverbindungen der Gruppe VIII des Periodensystems der Elemente, vorzugsweise Verbindungen des Kobalts, Rhodiums, Iridiums, Nickels, Eisens, Platins, Palladiums ode Rutheniums und insbesondere des Kobalts, Rhodiums und Iridiums. Besonders bevorzugt ist die Verwendung von Rhodium. Dabei werden im allgemeinen Rhodium-Verbindungen verwendet, die nicht mit phosphorhaltigen Liganden, wie Phosphinen oder Phosphiten modifiziert sind. Solche, nicht mit Phosphinen oder Phosphiten modifizierten Rhodiumkatalysatoren und ihre Eignung als Katalysator zur Hydroformylierung sind aus der Literatur bekannt und sie werden als unmodifizierte Rhodiumkatalysatoren bezeichnet. Es wird in der Fachliteratur angenommen, dass die Rhodium-Verbindung HRh(CO)₄ die katalytisch aktive Rhodiumspezies bei der Hydroformylierung mit unmodifizierten Rhodiumkatalysatoren ist, obgleich dies aufgrund der vielen in der Hydroformylierungszone nebeneinander ablaufenden Chemismen nicht eindeutig bewiesen ist. Da im allgemeinen die Verwendung von nicht mit Phosphinen modifizierten Rhodiumkatalysatoren einen geringeren Rhodiumgehalt erfordert, arbeitet man vorzugsweise in der zweiten Hydroformylierungsstufe mit unmodifizierten Rhodiumkatalysatoren. Der Rhodiumgehalt beträgt im allgemeinen von 5 bis 100 ppm, bezogen auf das homogene Reaktionsgemisch.

Man kann aber auch in der zweiten Hydroformylierungsstufe Rhodium-Komplexverbindungen verwenden, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von etwa 5 bis etwa 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphor-Liganden durchzuführen, der mit Rhodium keine Komplexverbindung mehr eingeht. Der freie Phosphor-Ligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylphenylphosphine, Cycloalkylphenylphosphine und organische Diphosphite. Wegen seiner leichten Zugänglichkeit wird Triphenylphosphin besonders häufig angewandt.

Arbeitet man mit einem modifizierten Rhodium-Komplexkatalysatorsystem, beträgt üblicherweise in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Verwendet man in der zweiten Hydroformylierungsstufe ein anderes Übergangsmetall der Gruppe VIII des Periodensystems der Elemente als Rhodium, so liegt die Konzentration an Übergangsmetall und das molare Verhältnis von Übergangsmetall zu Phosphor, falls man nach dem phosphinmodifizierten Verfahren arbeitet, in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Die Bedingungen, unter denen die Umsetzung in der zweiten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die zweite Hydroformylierungsstufe des rohen TCD-Monenal's bei Temperaturen von 70 bis 140°C durch. Bevorzugt hält man Temperaturen von 80 bis 130°C und insbesondere von 90 bis 120°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung und Synthesegas unter den Bedingungen der Hydroformylierungsreaktion im Reaktionsgemisch, gegebenenfalls in Gegenwart von organischen Phosphor(111)-Verbindungen. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators für die erste und zweite Reaktionsstufe gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, Co₂(CO)₈, Co₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet hat sich Rhodiumoxid, Rhodiumacetat, Rhodium-2-ethylhexanoat, Kobaltoxid, Kobaltacetat und Kobalt-2-ethylhexanoat erwiesen.

Die einzelnen Hydroformylierungsstufen können sowohl absatzweise als auch kontinuierlich durchgeführt werden.

Das Reaktionsprodukt der zweiten Hydroformylierungsstufe wird nach konventionellen Verfahren destilliert. Rhodium und gegebenenfalls in der zweiten Stufe zugesetzte organische Phosphorverbindungen fallen im Destillationsrückstand an und werden nach bekannten Methoden zurückgewonnen.

Das erfindungsgemäße Verfahren gestattet einen einfachen und kostengünstigen Zugang zu TCD-Dialdehyd in hoher Ausbeute und in hoher Reinheit. Der nach dem erfindungsgemäßen Verfahren hergestellte TCD-Dialdehyde lässt sich in ausgezeichneter Weise für unterschiedliche Anwendungen einsetzen.

Im folgenden wird das erfindungsgemäße Verfahren anhand einiger Beispiele näher erläutert, es ist jedoch nicht auf die beschriebenen Ausführungsformen beschränkt.

### Beispiele

Die bei der analytischen Charakterisierung der Reaktionsprodukte verwendeten Abkürzungen haben folgende Bedeutung:

| | |
|---|---|
| DCP | Dicyclopentadien |
| TCD-Monenal | 8(9)-Formyl-tricyclo[5.2.1.0^{2.6}]dec-3-en |
| TCD-Dial | 3(4),8(9)-Bisformyl-tricyclo[5.2.10^{2.6}]decan |
| Tri-CP | Tricyclopentadien. |
| | |
| TPPTS bedeutet | Natrium-Triphenylphosphintrisulfonat |

### Herstellung von TCD-Dialdehyd

### Beispiel 1

In einem 5 I-Autoklaven werden 2.119 g TPPTS-Lösung mit einem P(III)-Gehalt von 472 mmol/kg vorgelegt und mit 160,2 g Rh-Lösung (Rh-Gehalt: 6.423 mg/kg) versetzt. Danach wird eine Mischung aus 661,1 g Dicyclopentadien (technisch, DCP-Gehalt: 93,72 Gew.-%) und 283,0 g Toluol zugegeben. Das Reaktionsgemisch wird auf 135°C erwärmt und bei einem Synthesegasdruck von 2,5 MPa und einer Reaktionszeit von 6 Stunden umgesetzt.

Nach Reaktionsende wird abgekühlt und die obere, organische Phase von der wässrigen Katalysatorphase durch Phasentrennung abgetrennt. Die verbleibende Katalysatorphase wird erneut mit einem Gemisch aus Dicyclopentadien und Toluol versetzt und erneut umgesetzt. Dieser Vorgang wird insgesamt achtmal wiederholt.

Die organischen Phasen (Summe: 9.923 g) werden vereinigt und gaschromatographisch untersucht.

| GC-Analyse (in Fl.-%, Flächenprozent) | |
|---|---|
| Vorlaufkomponenten | 0,32 |
| Toluol | 29,45 |
| DCP | 4,55 |
| TCD-Monenal | 61,30 |
| TCD-Dial | 0,81 |
| Tri-CP | 0,42 |
| Sonstige | 3,15 |

Die Ausbeute an TCD-Monenal beträgt 91,6 %.

400,0 g rohes TCD-Monenal aus der ersten Reaktionsstufe werden ohne Anwendung weiterer Reinigungsschritte durch Zugabe einer toluolischen Lösung von Rh-2-ethylhexanoat auf einen Rhodiumgehalt von 20 ppm, bezogen auf die gesamte Reaktionslösung, eingestellt und in einem 1-I Autoklav vorgelegt. Das Reaktionsgemisch wird auf 120°C erwärmt und bei einem Druck von 26,0 MPa und einer Reaktionszeit von 6 Stunden umgesetzt. Nach Reaktionsende wird abgekühlt und entspannt, das erhaltene Reaktionsprodukt (455,9 g) wird gaschromatographisch untersucht.

| GC-Analyse (in Fl.-%) | |
|---|---|
| Vorlaufkomponenten | 1,30 |
| Toluol | 31,70 |
| TCD-Monenal | 2,32 |
| TCD-Dial | 62,36 |
| Sonstige | 2,32 |

Die Ausbeute an TCD-Dialdehyd liegt bei 94,5 % d.Th.

Zur Aufarbeitung wird der nach der zweiten Hydroformylierungsstufe erhaltene Dialdehyd (450 g) an einer Claisenbrücke destilliert. Man erhält 303,2 g Destillat in einem Siedebereich von 115 - 142°C bei einem Druck von 2 hPa mit folgender Zusammensetzung:

| GC-Analyse (in Fl.-%) | |
|---|---|
| Vorlaufkomponenten | 0,17 |
| TCD-Monenal | 1,02 |
| TCD-Dial | 98,27 |
| Sonstige | 0,54 |

Daraus ergibt sich eine Destillationsausbeute an TCD-Dialdehyd von 95,1 %, bezogen auf eingesetzten, rohen TCD-Dialdehyd. Die Rückstandsmenge beträgt 11,8 g (2,6 Gew.-% vom Destillationseinsatz).

### Beispiel 2

Analog Beispiel 1 werden 400 g TCD-Monenal (Zusammensetzung wie in Beispiel 1) in Gegenwart von 30 ppm Rh bei einer Temperatur von 100°C und Reaktionszeiten von 6 und 8 Stunden umgesetzt. Nach Reaktionsende werden 452,9 g (6 Stunden) und 458,7 g (8 Stunden) Oxorohprodukt erhalten und gaschromatographisch untersucht.

| Reaktionszeit (Std.) | 6 | 8 |
|---|---|---|
| | | |
| GC-Analyse (in Fl.-%) | | |
| | | |
| Vorlaufkomponenten | 1,04 | 1,45 |
| Toluol | 30,60 | 31,15 |
| TCD-Monenal | 5,90 | 3,10 |
| TCD-Dial | 61,07 | 62,58 |
| Sonstige | 1,39 | 1,72 |
| | | |
| Ausbeute (%) | 90,3 | 94,9 |

Der gemäß Beispiel 1 bestimmte Destillationsrückstand liegt bei 2,4 Gew.-% (6 Stunden Reaktionszeit) und 2,7 Gew.-% (8 Stunden Reaktionszeit) vom Destillationseinsatz.

### Beispiel 3

Analog Beispiel 1 werden 400 g TCD-Monenal (Zusammensetzung wie in Beispiel 1) in Gegenwart von 30 ppm Rh bei einer Temperatur von 110°C und Reaktionszeiten von 6 und 8 Stunden umgesetzt. Nach Reaktionsende werden 454,9 g (6 Stunden) und 456,7 g (8 Stunden) Oxorohprodukt erhalten und gaschromatographisch untersucht.

| Reaktionszeit (Std.) | 6 | 8 |
|---|---|---|
| | | |
| GC-Analyse (in Fl.-%) | | |
| | | |
| Vorlaufkomponenten | 1,28 | 1,34 |
| Toluol | 32,50 | 33,20 |
| TCD-Monenal | 3,24 | 2,94 |
| TCD-Dial | 61,36 | 60,85 |
| Sonstige | 1,62 | 1,67 |
| | | |
| Ausbeute (%) | 93,8 | 94,6 |

Der gemäß Beispiel 1 bestimmte Destillationsrückstand liegt bei 2,6 Gew.-% (6 Stunden Reaktionszeit) und 2,9 Gew.-% (8 Stunden Reaktionszeit) vom Destillationseinsatz.

### Beispiel 4

Analog Beispiel 1 werden 400 g TCD-Monenal (Zusammensetzung wie in Beispiel 1) in Gegenwart von 30 ppm Rh bei einer Temperatur von 120°C und Reaktionszeiten von 4 und 6 Stunden umgesetzt. Nach Reaktionsende werden 457,1 g (4 Stunden) und 458,1 g (6 Stunden) Oxorohprodukt erhalten und gaschromatographisch untersucht.

| Reaktionszeit (Std.) | 4 | 6 |
|---|---|---|
| | | |
| GC-Analyse (in Fl.-%) | | |
| | | |
| Vorlaufkomponenten | 1,22 | 1,10 |
| Toluol | 32,30 | 31,55 |
| TCD-Monenal | 3,86 | 3,70 |
| TCD-Dial | 61,40 | 62,22 |
| Sonstige | 1,22 | 1,43 |
| Ausbeute (%) | 94,2 | 94,7 |

Der gemäß Beispiel 1 bestimmte Destillationsrückstand liegt bei 2,9 Gew.-% (4 Stunden Reaktionszeit) und 3,2 Gew.-% (6 Stunden Reaktionszeit) vom Destillationseinsatz.

### Beispiel 5

Analog Beispiel 1 werden 400 g TCD-Monenal (Zusammensetzung wie in Beispiel 1) in Gegenwart von 20 ppm Rh bei einer Temperatur von 110°C und Reaktionszeiten von 6 und 8 Stunden umgesetzt. Nach Reaktionsende werden 456,5 g (6 Stunden) und 457,9 g (8 Stunden) Oxorohprodukt erhalten und gaschromatographisch untersucht.

| Reaktionszeit (Std.) | 6 | 8 |
|---|---|---|
| | | |
| GC-Analyse (in Fl.-%) | | |
| | | |
| Vorlaufkomponenten | 1,31 | 1,41 |
| Toluol | 31,25 | 32,75 |
| TCD-Monenal | 5,50 | 3,77 |
| TCD-Dial | 60,36 | 61,05 |
| Sonstige | 1,58 | 1,02 |
| | | |
| Ausbeute (%) | 91,1 | 94,5 |

Der gemäß Beispiel 1 bestimmte Destillationsrückstand liegt bei 2,7 Gew.-% (6 Stunden Reaktionszeit) und 3,0 Gew.-% (8 Stunden Reaktionszeit) vom Destillationseinsatz.

### Beispiel 6

Analog Beispiel 1 werden 400 g TCD-Monenal (Zusammensetzung wie in Beispiel 1) in Gegenwart von 20 ppm Rh bei einer Temperatur von 120°C und einer Reaktionszeit von 8 Stunden umgesetzt. Nach Reaktionsende werden 457,2 g Oxorohprodukt erhalten und gaschromatographisch untersucht.

| GC-Analyse (in Fl.-%) | |
|---|---|
| | |
| Vorlaufkomponenten | 1,35 |
| Toluol | 32,55 |
| TCD-Monenal | 2,09 |
| TCD-Dial | 61,65 |
| Sonstige | 2,36 |
| | |
| Ausbeute (%) | 95,0 |

Der gemäß Beispiel 1 bestimmte Destillationsrückstand liegt bei 2,3 Gew.-% vom Destillationseinsatz.

### Beispiel 7

Gemäß Beispiel 1 werden 400 g TCD-Monenal folgender Zusammensetzung in Gegenwart von 30 ppm Rh bei einer Temperatur von 110°C und einer Reaktionszeit von 6 Stunden umgesetzt.

| GC-Analyse (in Fl.-%) | |
|---|---|
| | |
| Vorlaufkomponenten | 0,85 |
| Toluol | 0,70 |
| DCP | 2,84 |
| TCD-Monenal | 89,10 |
| TCD-Dial | 1,49 |
| Tri-CP | 0,60 |
| Sonstige | 4,42 |

Nach Reaktionsende werden 465,0 g Oxorohprodukt erhalten und gaschromatographisch untersucht.

| GC-Analyse (in Fl.-%) | |
|---|---|
| | |
| Vorlaufkomponenten | 1,78 |
| Toluol | 1,15 |
| TCD-Monenal | 4,79 |
| TCD-Dial | 89,96 |
| Sonstige | 2,32 |
| | |
| Ausbeute (%) | 94,0 |

Der gemäß Beispiel 1 bestimmte Destillationsrückstand liegt bei 2,1 Gew.-% vom Destillationseinsatz.

### Beispiel 8

Gemäß Beispiel 1 werden 400 g TCD-Monenal folgender Zusammensetzung in Gegenwart von 30 ppm Rh bei einer Temperatur von 120°C und einer Reaktionszeit von 8 Stunden umgesetzt.

| GC-Analyse (in Fl.-%) | |
|---|---|
| | |
| Vorlaufkomponenten | 0,85 |
| Toluol | 0,70 |
| DCP | 2,84 |
| TCD-Monenal | 89,10 |
| TCD-Dial | 1,49 |
| Tri-CP | 0,60 |
| Sonstige | 4,42 |

Nach Reaktionsende werden 467,5 g Oxorohprodukt erhalten und gaschromatographisch untersucht.

| GC-Analyse (in Fl.-%) | |
|---|---|
| | |
| Vorlaufkomponenten | 1,55 |
| Toluol | 1,51 |
| TCD-Monenal | 3,34 |
| TCD-Dial | 90,58 |
| Sonstige | 3,02 |
| | |
| Ausbeute (%) | 95,2 |

Der gemäß Beispiel 1 bestimmte Destillationsrückstand liegt bei 1,9 Gew.-% vom Destillationseinsatz.

### Beispiel 9

Gemäß Beispiel 1 werden je 400 g TCD-Monenal folgender Zusammensetzung in Gegenwart von 20 ppm Rh bei einer Temperatur von 110°C und einer Reaktionszeit von 8 Stunden umgesetzt.

| GC-Analyse Einsatzprodukte (in Fl.-%) | | | | | | |
|---|---|---|---|---|---|---|
| | | | | | | |
| Vorlaufkomponenten | 0,02 | 0,19 | 0,24 | 0,29 | 0,36 | 0,41 |
| Toluol | 29,61 | 23,08 | 21,01 | 22,45 | 20,76 | 21,10 |
| DCP | 9,01 | 11,84 | 16,97 | 24,45 | 34,81 | 43,21 |
| TCD-Monenal | 59,29 | 62,94 | 60,00 | 50,99 | 42,52 | 33,89 |
| TCD-Dial | 1,49 | 1,37 | 1,41 | 1,27 | 1,07 | 0,85 |
| Tri-CP | 0,38 | 0,46 | 0,23 | 0,27 | 0,27 | 0,34 |
| Sonstige | 0,20 | 0,12 | 0,14 | 0,28 | 0,21 | 0,20 |

| GC-Analyse Reaktionsprodukte (in Fl.-%) | | | | | | |
|---|---|---|---|---|---|---|
| | | | | | | |
| VorlaufKomponenten | 0,48 | 0,42 | 0,59 | 0,32 | 0,37 | 0,49 |
| Toluol | 27,82 | 21,88 | 20,41 | 23,95 | 24,26 | 26,25 |
| TCD-Monenal | 3,15 | 4,20 | 4,13 | 4,33 | 3,11 | 2,96 |
| TCD-Dial | 68,10 | 73,03 | 74,26 | 70,98 | 71,71 | 69,25 |
| Sonstige | 0,45 | 0,47 | 0,61 | 0,42 | 0,55 | 0,93 |
| Destillationsrückstand (Gew.-%) vom Destillationseinsatz | 4,9 | 6,9 | 9,5 | 14,9 | 17,1 | 22,5 |

Aus diesen Versuchen kann man ersehen, dass die Rückstandsmengen mit zunehmendem DCP-Gehalt ansteigen, die Reaktionsprodukte aus der DCP-Hydroformylierung unter Rh/TPPTS-Katalyse aber unabhängig vom DCP-Gehalt in die zweite Hydroformylierungsstufe eingesetzt werden können. Trotz eines hohen DCP-Gehalts in dem TCD-Monenal führt die Umsetzung in der zweiten Reaktionsstufe nur zu einem geringen Restgehalt an TCD-Monenal.

## Patentansprüche

1. Verfahren zur Herstellung von 3(4),8(9)-Bisformyl-tricyclo[5.2.1.0^{2.6}]decan durch Hydroformylierung von Dicyclopentadien mit nachfolgender Destillation, **dadurch gekennzeichnet, dass** man Dicyclopentadien in einer ersten Hydrofomylierungsstufe in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas zum 8(9)-Formyl-tricyclo[5.2.1.0^{2.6}]dec-3-en umsetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Übergangsmetallverbindungen der Gruppe VIII des Periodensystems der Elemente bei Temperaturen von 70 bis 140°C und Drücken von 5 bis 35 MPa durch Umsetzung mit Synthesegas in 3(4),8(9)-Bisformyl-tricyclo[5.2.1.0^{2.6}]decan überführt und das so erhaltene 3(4),8(9)-Bisformyl-tricyclo[5.2.1.0^{2.6}]decan anschließend destilliert.

2. Verfahren gemäß Anspruch 1, **dadurch gekennzeichnet, dass** das in der ersten Hydroformylierungsstufe erhaltene 8(9)-Formyl-tricyclo[5.2.1.0^{2.6}]dec-3-en vor dem Einsatz in die zweite Hydroformylierungsstufe destilliert wird.

3. Verfahren gemäß Anspruch 1 oder 2, **dadurch gekennzeichnet, dass** in der zweiten Hydroformylierungsstufe die Umsetzung in Gegenwart von organischen Phosphor(III)-Verbindungen erfolgt.

4. Verfahren gemäß Anspruch 3, **dadurch gekennzeichnet, dass** man als organische Phosphor(III)-Verbindungen Triarylphosphine, Trialkylphosphine, Alkylphenylphosphine, Cycloalkylphenylphosphine und organische Diphosphite verwendet.

5. Verfahren gemäß einem oder mehreren der Anspruch 1 bis 4, **dadurch gekennzeichnet, dass** in der ersten Hydroformylierungsstufe als wasserlösliche organische Phoshor(III)-Verbindungen sulfonierte Triarylphosphine der allgemeinen Formel (1) 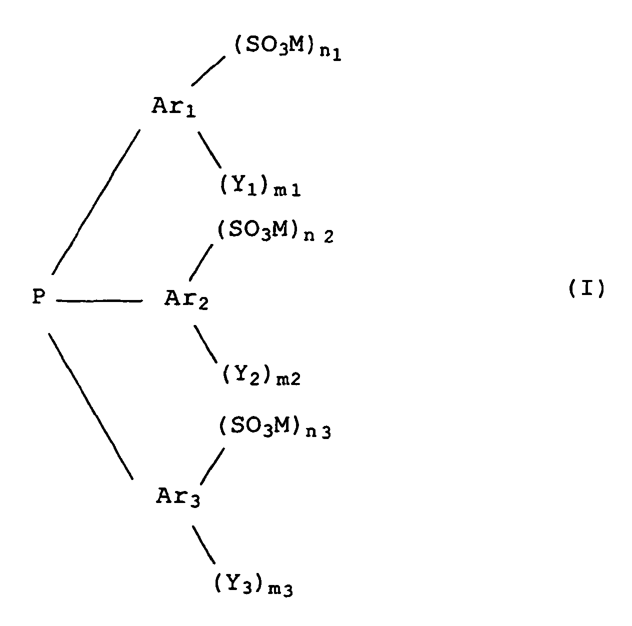 eingesetzt werden, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

6. Verfahren gemäß Anspruch 5, **dadurch gekennzeichnet, dass** Ar₁, Ar₂, Ar₃ jeweils eine Phenylgruppe bedeuten, m₁, m₂, m₃ gleich 0 sind, n₁, n₂, n₃ gleich 0 oder 1 sind und n₁+n₂+n₃ zusammen 1 bis 3 ausmachen, und dass die Sulfonat-Gruppen in meta-Stellung stehen.

7. Verfahren gemäß einem oder mehreren der Ansprüche 1 bis 4, **dadurch gekennzeichnet, dass** in der ersten Hydroformylierungsstufe als wasserlösliche organische Phosphor(III)-Verbindungen sulfonierte Diphosphine der allgemeinen Formel (II) 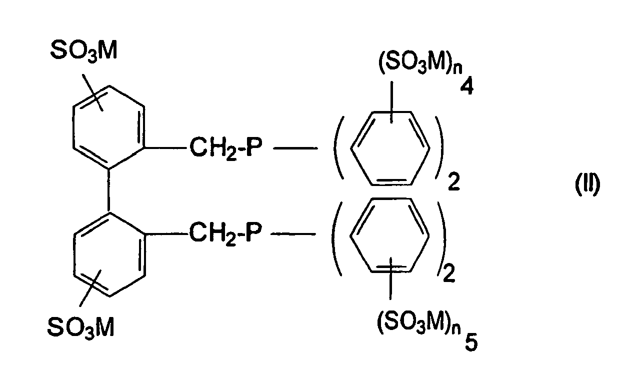 in der n₄ und n₅ unabhängig voneinander für 0 oder 1 steht, wobei die sulfonierten Diphosphine der allgemeinen Formel (II) bis zu sechs SO₃M-Gruppen enthalten, und M Ammonium, ein einwertiges Metall oder das Äquivalent eines mehrwertigen Metalls bedeutet.

8. Verfahren gemäß einem oder mehreren der Ansprüche 1 bis 4, **dadurch gekennzeichnet, dass** in der ersten Hydroformylierungsstufe als wasserlösliche organische Phosphor(III)-Verbindungen sulfonierte Diphosphine der allgemeinen Formel (III) 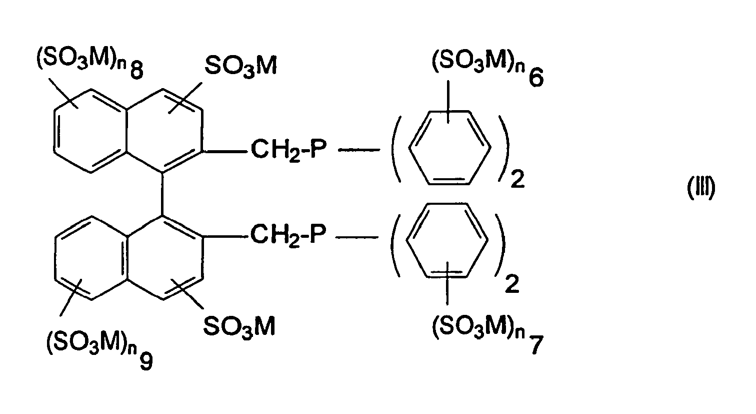 in der n₆, n₇, n₈ und n₉ unabhängig voneinander für 0 oder 1 steht, wobei die sulfonierten Diphosphine der allgemeinen Formel (111) vier bis acht SO₃M-Gruppen enthalten, und M Ammonium, ein einwertiges Metall oder das Äquivalent eines mehrwertigen Metalls bedeutet.

9. Verfahren gemäß einem oder mehreren der Ansprüche 1 bis 8, **dadurch gekennzeichnet, dass** in der ersten Hydroformylierungsstufe als Obergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

10. Verfahren gemäß einem oder mehreren der Ansprüche 1 bis 8, **dadurch gekennzeichnet, dass** in der zweiten Hydroformylierungsstufe als Übergangsmetallverbindungen der Gruppe VIII des Periodensystems der Elemente Verbindungen von Rhodium, Kobalt, Iridium, Nickel, Platin, Palladium, Eisen oder Ruthenium verwendet werden.

11. Verfahren gemäß einem oder mehreren der Ansprüche 1 bis 10, **dadurch gekennzeichnet, das** in der ersten und zweiten Hydroformylierungsstufe als Übergangsmetallverbindungen der Gruppe VIII des Periodensystems der Elemente Verbindungen von Rhodium eingesetzt werden.

12. Verfahren gemäß einem oder mehreren der Ansprüche 1 bis 11, **dadurch gekennzeichnet, dass** in der ersten Hydroformylierungsstufe die Temperatur 100 bis 150°C, bevorzugt 110 bis 140°C, und der Druck 1 bis 6 MPa, bevorzugt 1,5 bis 5 MPa, beträgt.

13. Verfahren gemäß einem oder mehreren der Ansprüche 1 bis 12, **dadurch gekennzeichnet, dass** in der zweiten Hydroformylierungsstufe die Temperatur 80 bis 130°C, bevorzugt 90 bis 120°C, und der Druck 10 bis 30 MPa, bevorzugt 20 bis 30 MPa beträgt.

14. Verfahren gemäß einem oder mehreren der Ansprüche 1 bis 13, **dadurch gekennzeichnet, dass** in der ersten Hydroformylierungsstufe die Rhodium-Konzentration 20 bis 1000 Gew.-ppm, vorzugsweise 50 bis 800 Gew.-ppm und insbesondere 100 bis 600 Gew.-ppm, jeweils bezogen auf die wässrige Katalysatorlösung, beträgt.

15. Verfahren zur Herstellung gemäß einem oder mehreren der Ansprüche 1 bis 14, **dadurch gekennzeichnet, dass** in der ersten Hydroformylierungsstufe je mol Rhodium 10 bis 300 mol, insbesondere 50 bis 150 mol, Phosphor in Form der wasserlöslichen organischen Phosphorverbindung verwendet werden.

16. Verfahren gemäß einem oder mehreren der Ansprüche 1, 2, 5 bis 15, **dadurch gekennzeichnet, dass** in der zweiten Hydroformylierungsstufe die Rhodium-Konzentration 5 bis 100 Gew.-ppm, bezogen auf das homogene Reaktionsgemisch, beträgt.

17. Verfahren gemäß einem oder mehreren der Ansprüche 3 bis 15, **dadurch gekennzeichnet, dass** in der zweiten Hydroformylierungsstufe die Rhodium-Konzentration 5 bis 1000 Gew.-ppm, vorzugsweise 10 bis 700 Gew.-ppm und insbesondere 20 bis 500 Gew.-ppm, jeweils bezogen auf das homogene Reaktionsgemisch, beträgt und je mol Rhodium 5 bis 200 mol, vorzugsweise 10 bis 100 mol Phosphor in Form organischer Phosphorverbindungen verwendet werden.

## Claims

1. A process for preparing 3(4),8(9)-bisformyltricyclo[5.2.1.0^{2,6}]decane by hydroformylating dicyclopentadiene with subsequent distillation, which comprises reacting dicyclopentadiene, in a first hydroformylation stage in a heterogeneous reaction system using an aqueous solution of transition metal compounds, containing water-soluble organic phosphorus (III) compounds in complex-bound form, of group VIII of the Periodic Table of the Elements at temperatures of from 70 to 150°C and pressures of from 0.5 to 10 MPa, with synthesis gas to give 8(9)-formyltricyclo[5.2.1.0^{2,6}]dec-3-ene, then separating the organic phase from the aqueous phase and subsequently converting the thus obtained 8(9)-formyltricyclo[5.2.1.0²'⁶]dec-3-ene, in a second hydroformylation stage in homogeneous organic phase in the presence of transition metal compounds of group VIII of the Periodic Table of the Elements at temperatures of from 70 to 140°C and pressures of from 5 to 35 MPa by reacting with synthesis gas, to 3(4),8(9)-bisformyltricyclo[5.2.1.0^{2,6}]decane and subsequently distilling the thus obtained 3(4),8(9)-bisformyltricyclo[5.2.1.0^{2,6}]decane.

2. The process as claimed in claim 1, wherein 8(9)-formyltricyclo[5.2.1.0^{2,6}]dec-3-ene obtained in the first hydroformylation stage is distilled before use in the second hydroformylation stage.

3. The process as claimed in claim 1 or 2, wherein, in the second hydroformylation stage, the reaction is effected in the presence of organic phosphorus (III) compounds.

4. The process as claimed in claim 3, wherein the organic phosphorus (III) compounds used are triarylphosphines, trialkylphosphines, alkylphenylphosphines, cycloalkylphenylphosphines and organic diphosphites.

5. The process as claimed in one or more of claims 1 to 4, wherein the water-soluble organic phosphorus (III) compounds used in the first hydroformylation stage are sulfonated triarylphosphines of the general formula (1) 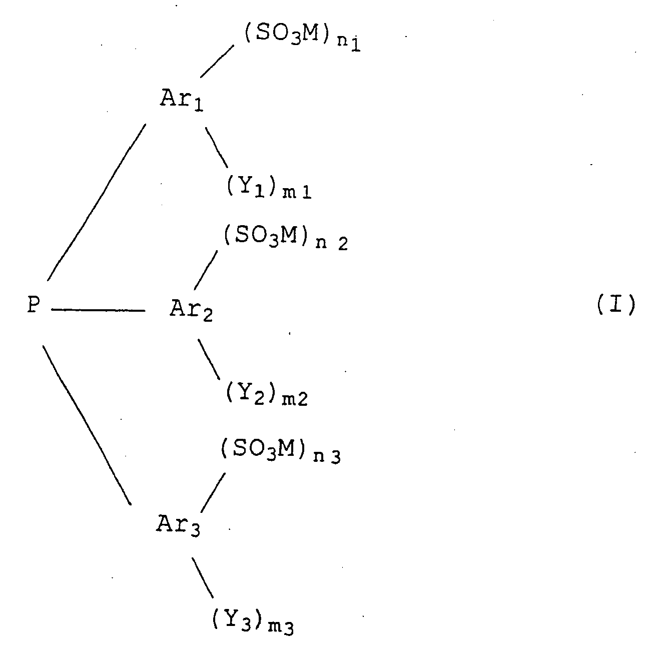 in which Ar¹, Ar² and Ar³ are identical or different aryl groups having from 6 to 14 carbon atoms, the substituents Y₁, Y₂ and Y₃ are identical or different, straight-chain or branched alkyl or alkoxy radicals having from 1 to 4 carbon atoms, chlorine, bromine, the hydroxyl, cyanide or nitro group, and also the amino group of the formula NR¹R² in which the substituents R¹ and R² are the same or different and are each hydrogen, straight-chain or branched alkyl groups having from 1 to 4 carbon atoms, in which M is lithium, sodium, potassium, magnesium, calcium or barium, in which m₁, m₂ and m₃ are the same or different and are each integers from 0 to 5, in which n₁. n₂ and n₃ are the same or different and are each integers from 0 to 3, and at least one of the numbers n₁, n₂ and n₃ is equal to or greater than 1.

6. The process as claimed in claim 5, wherein Ar₁. Ar₂, Ar₃ are each a phenyl group, m₁, m₂, m₃ are each 0, n₁, n₂, n₃ are each 0 or 1 and n₁+n₂+n₃ together add up to from 1 to 3, and the sulfonate groups are in the meta-position.

7. The process as claimed in one or more of claims 1 to 4, wherein the water-soluble organic phosphorus (III) compounds used in the first hydroformylation stage are sulfonated diphosphines of the general formula (II) 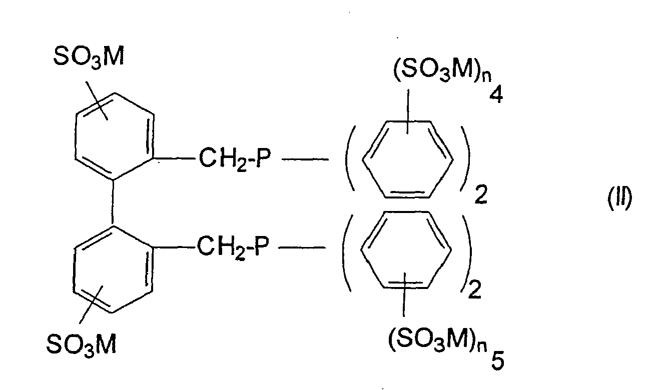 in which n₄ and n₅ are each independently 0 or 1, and the sulfonated diphosphines of the general formula (II) contain up to six SO₃M groups, and M is ammonium, a monovalent metal or the equivalent of a polyvalent metal.

8. The process as claimed in one or more of claims 1 to 4, wherein the water-soluble organic phosphorus (III) compounds used in the first hydroformylation stage are sulfonated diphosphines of the general formula (III) 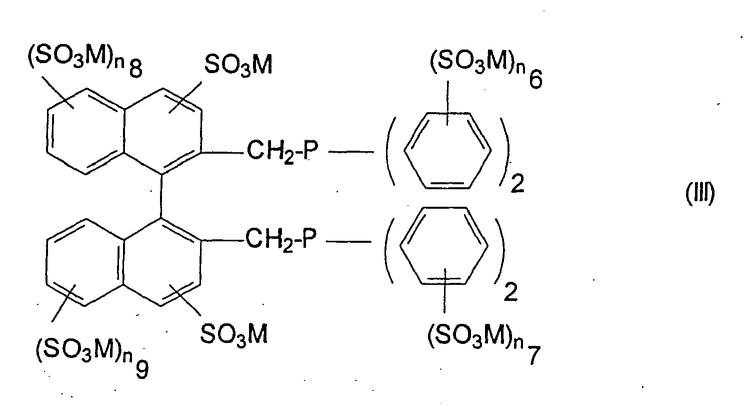 in which n₆, n₇, n₈ and n₉ are each independently 0 or 1, and the sulfonated diphosphines of the general formula (III) contain from four to eight SO₃M groups, and M is ammonium, a monovalent metal or the equivalent of a polyvalent metal.

9. The process as claimed in one or more of claims 1 to 8, wherein the transition metal compounds, used in the first hydroformylation stage, of group VIII of the Periodic Table of the Elements are compounds of rhodium, cobalt, iridium, nickel, palladium, platinum, iron or ruthenium.

10. The process as claimed in one or more of claims 1 to 8, wherein the transition metal compounds, used in the second hydroformylation stage, of group VIII of the Periodic Table of the Elements are compounds of rhodium, cobalt, iridium, nickel, platinum, palladium, iron or ruthenium.

11. The process as claimed in one or more of claims 1 to 10, wherein the transition metal compounds, used in the first and second hydroformylation stage, of group VIII of the Periodic Table of the Elements are compounds of rhodium.

12. The process as claimed in one or more of claims 1 to 11, wherein the temperature in the first hydroformylation stage is from 100 to 150°C, preferably from 110 to 140°C, and the pressure is from 1 to 6 MPa, preferably from 1.5 to 5 MPa.

13. The process as claimed in one or more of claims 1 to 12, wherein the temperature in the second hydroformylation stage is from 80 to 130°C, preferably from 90 to 120°C, and the pressure is from 10 to 30 MPa, preferably from 20 to 30 MPa.

14. The process as claimed in one or more of claims 1 to 13, wherein the rhodium concentration in the first hydroformylation stage is from 20 to 1000 ppm by weight, preferably from 50 to 800 ppm by weight and especially from 100 to 600 ppm by weight, based in each case on the aqueous catalyst solution.

15. The process for the preparation as claimed in one or more of claims 1 to 14, wherein from 10 to 300 mol, especially from 50 to 150 mol, of phosphorus in the form of the water-soluble organic phosphorus compound are used per mole of rhodium in the first hydroformylation stage.

16. The process according to one or more of claims 1, 2, 5 to 15, wherein the rhodium concentration in the second hydroformylation stage is from 5 to 100 ppm by weight, based on the homogeneous reaction mixture.

17. The process according to one or more of claims 3 to 15, wherein the rhodium concentration in the second hydroformylation stage is from 5 to 1000 ppm by weight, preferably from 10 to 700 ppm by weight and especially from 20 to 500 ppm by weight, based in each case on the homogeneous reaction mixture, and from 5 to 200 mol, preferably from 10 to 100 mol, of phosphorus in the form of organic phosphorus compounds are used per mole of rhodium.

## Revendications

1. Procédé de préparation du 3(4),8(9)-bisformyl-tricyclo[5.2.1.0^{2,6}]décane par hydroformylation du dicyclopentadiène, suivie d'une distillation, **caractérisé en ce qu'**on fait réagir du dicyclopentadiène, dans une première étape d'hydroformylation dans un système réactionnel hétérogène, par utilisation d'une solution aqueuse de composés d'un métal de transition du Groupe VIII du Tableau Périodique des Eléments, contenant en liaison complexe des composés organiques solubles dans l'eau du phosphore(III), à des températures de 70 à 150°C et sous des pressions de 0,5 à 10 MPa, avec un gaz de synthèse pour obtenir le 8(9)-formyl-tricyclo[5.2.1.0^{2,6}] dec-3-ène, ce après quoi on sépare la phase organique de la phase aqueuse, puis on convertit le 8(9)-formyl-tricyclo[5.2.1.0^{2,6}]dec-3-ène ainsi obtenu, dans une deuxième étape d'hydroformylation en phase organique homogène, en présence de composés de métaux de transition du Groupe VIII du Tableau Périodique des Eléments, à des températures de 70 à 140°C et sous des pressions de 5 à 35 MPa, par réaction avec un gaz de synthèse, en 3(4),8(9)-bisformyl-tricyclo[5.2.1.0^{2,6}]décane, puis on distille le 3(4),8(9)-bisformyl-tricyclo[5.2.1.0^{2,6}] décane ainsi obtenu.

2. Procédé selon la revendication 1, **caractérisé en ce que** le 8(9)-formyl-tricyclo[5.2.1.0^{2,6}]dec-3-ène obtenu dans la première étape d'hydroformylation est distillé avant d'être utilisé dans la deuxième étape d'hydroformylation.

3. Procédé selon la revendication 1 ou 2, **caractérisé en ce que** la réaction, dans la deuxième étape d'hydroformylation, a lieu en présence de composés organiques du phosphore(III).

4. Procédé selon la revendication 3, **caractérisé en ce qu'**on utilise en tant que composés organiques du phosphore(III) des triarylphosphines, des trialkylphosphines, des alkylphénylphosphines, des cycloalkylphénylphosphines et des diphosphites organiques.

5. Procédé selon l'une ou plusieurs des revendications 1 à 4, **caractérisé en ce que**, dans la première étape d'hydroformylation, on utilise en tant que composés organiques du phosphore(III) solubles dans l'eau des triarylphosphines sulfonées, de formule générale (I) 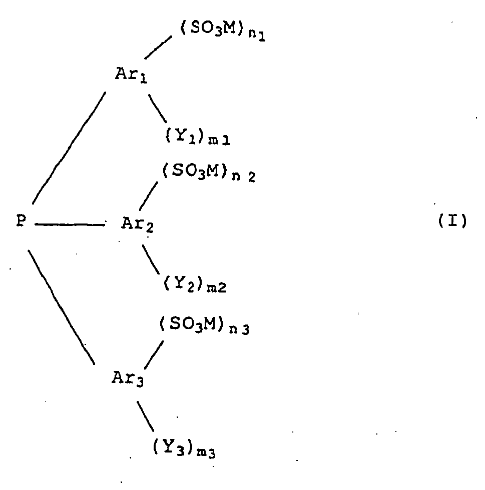 dans laquelle Ar¹, Ar² et Ar³ sont des groupes aryle identiques ou différents ayant 6 à 14 atomes de carbone, les substituants Y₁, Y₂ et Y₃ sont des radicaux alkyle ou alcoxy identiques ou différents, à chaîne droite ou ramifiée, ayant 1 à 4 atomes de carbone, des atomes de chlore, de brome, ou encore des groupes hydroxyle, cyanure ou nitro, ou encore représentent le groupe amino de formule NR¹R², dans laquelle les substituants R¹ et R² sont identiques ou différents et représentent chacun un atome d'hydrogène, un groupe alkyle à chaîne droite ou ramifiée ayant 1 à 4 atomes de carbone, M étant le lithium, le sodium, le potassium, le magnésium, le calcium ou le baryum, et m₁, m₂ et m₃ sont identiques ou différents et représentent des nombres de 0 à 5 ; n₁, n₂ et n₃ sont identiques ou différents et représentent des nombres entiers de 0 à 3, au moins l'un des nombres n₁, n₂ et n₃ étant supérieur ou égal à 1.

6. Procédé selon la revendication 5, **caractérisé en ce que** chacun des radicaux Ar₁, Ar₂ et Ar₃ est un groupe phényle ; m₁, m₂ et m₃ valent chacun 0 ; n₁, n₂ et n₃ valent chacun 0 ou 1 ; et n1+n2+n3 vaut 1 à 3, et **en ce que** les groupes sulfonate sont en position méta.

7. Procédé selon l'une ou plusieurs des revendications 1 à 4, **caractérisé en ce que**, dans la première étape d'hydroformylation, on utilise en tant que composés organiques du phosphore(III) solubles dans l'eau des diphosphines sulfonées de formule générale (II) 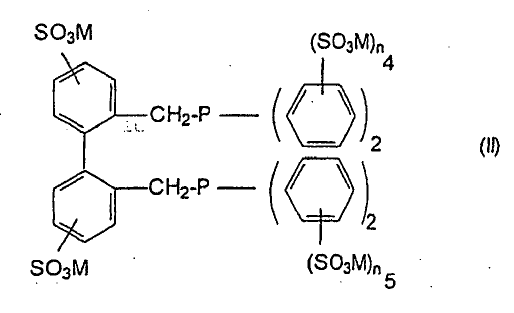 dans laquelle n₄ et n₅ valent chacun indépendamment de l'autre 0 ou 1, les diphosphines sulfonées de formule générale (II) contenant jusqu'à six groupes SO₃M, et M représente l'ammonium, un métal monovalent ou l'équivalent d'un métal polyvalent.

8. Procédé selon l'une ou plusieurs des revendications 1 à 4, **caractérisé en ce que**, dans la première étape d'hydroformylation, on utilise en tant que composés organiques du phosphore(III) solubles dans l'eau des diphosphines sulfonées de formule générale (III) 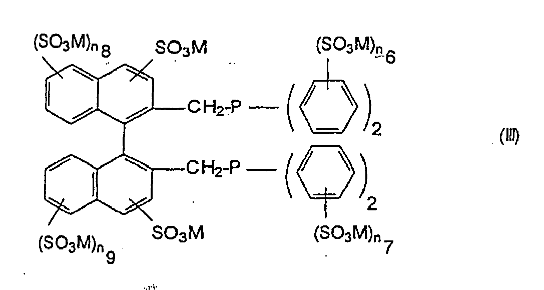 dans laquelle n₆, n₇, n₈ et n₉ valent chacun indépendamment des autres 0 ou 1, les diphosphines sulfonées de formule générale (III) contenant 4 à 8 groupes SO₃M, et M représentant l'ammonium, un métal monovalent ou l'équivalent d'un métal polyvalent.

9. Procédé selon l'une ou plusieurs des revendications 1 à 8, **caractérisé en ce que**, dans la première étape d'hydroformylation, on utilise en tant que composés d'un métal de transition du Groupe VIII du Tableau Périodique des Eléments des composés du rhodium, du cobalt, de l'iridium, du nickel, du palladium, du platine, du fer ou du ruthénium.

10. Procédé selon l'une ou plusieurs des revendications 1 à 8, **caractérisé en ce que**, dans la deuxième étape d'hydroformylation, on utilise en tant que composés d'un métal de transition du Groupe VIII du Tableau Périodique des Eléments des composés du rhodium, du cobalt, de l'iridium, du nickel, du platine, du palladium, du fer ou du ruthénium.

11. Procédé selon l'une ou plusieurs des revendications 1 à 10, **caractérisé en ce que**, dans la première et dans la deuxième étapes d'hydroformylation, on utilise en tant que composés d'un métal de transition du Groupe VIII du Tableau Périodique des Eléments des composés du rhodium.

12. Procédé selon l'une ou plusieurs des revendications 1 à 11, **caractérisé en ce que**, dans la première étape d'hydroformylation, la température est de 100 à 150°C, de préférence de 110 à 140°C, et la pression est de 1 à 6 MPa, de préférence de 1,5 à 5 MPa.

13. Procédé selon l'une ou plusieurs des revendications 1 à 12, **caractérisé en ce que**, dans la deuxième étape d'hydroformylation, la température est de 80 à 130°C, de préférence de 90 à 120°C, et la pression est de 10 à 30 MPa, de préférence de 20 à 30 MPa.

14. Procédé selon l'une ou plusieurs des revendications 1 à 13, **caractérisé en ce que**, dans la première étape d'hydroformylation, la concentration du rhodium est de 20 à 1 000 ppm en poids, de préférence de 50 à 800 ppm en poids et en particulier de 100 à 600 ppm en poids, dans tous les cas par rapport à la solution aqueuse de catalyseur.

15. Procédé de préparation selon l'une ou plusieurs des revendications 1 à 14, **caractérisé en ce que**, dans la première étape d'hydroformylation, on utilise par mole de rhodium 10 à 300 moles, en particulier 50 à 150 moles de phosphore sous forme du composé organophosphoré soluble dans l'eau.

16. Procédé selon l'une ou plusieurs des revendications 1, 2, 5 à 15, **caractérisé en ce que**, dans la deuxième étape d'hydroformylation, la concentration du rhodium est de 5 à 100 ppm en poids par rapport au mélange réactionnel homogène.

17. Procédé selon l'une ou plusieurs des revendications 3 à 15, **caractérisé en ce que**, dans la deuxième étape d'hydroformylation, la concentration du rhodium est de 5 à 1 000 ppm en poids, de préférence de 10 à 700 ppm en poids et en particulier de 20 à 500 ppm en poids, dans tous les cas par rapport au mélange réactionnel homogène, et que l'on utilise par mole de rhodium 5 à 200 moles, de préférence 10 à 100 moles de phosphore sous forme de composés organophosphorés.
